# EUROPEAN PATENT APPLICATION

(11) **EP 2 942 624 A1**
(43) Date of publication of application: **11.11.2015**
(21) Application number: 14176323.5
(22) Date of filing: 09.07.2014
(51) Int. Cl.: G01N 33/49, A61M 1/34, B01D 61/14

(54) **Device and method for extracting concentrated plasma**

(30) Priority: 09.05.2014 KR 20140055596
(71) Applicant: Kim, Hong, Asan City, Chungcheongnam-do (KR)
(72) Inventor: Kim, Hong, Asan City, Chungcheongnam-do (KR)
(74) Representative: Kuhnen & Wacker

(57) **Abstract**

The present invention relates to a device and method for extracting concentrated plasma that includes a membrane filter unit for isolating a component based on a predetermined particle diameter from blood plasma, a first receiving member for providing a predetermined injection rate and a predetermined injection pressure to inject the blood plasma into the membrane filter unit and thereby to settle down platelets in the blood plasma, a pressurizing means mounted in connection to the first receiving member so as to provide an injection rate and an injection pressure for the blood plasma, and a second receiving member mounted on the membrane filter unit at a predetermined distance from the first receiving member and open into a second space to hold air, where the second receiving member is allowed to open or close. This can pass the blood plasma through the membrane filter unit for a sufficient period of time using a predetermined pressure, gravity and vacuum, thereby maximizing the settlement of the platelets in the blood plasma flowing into the membrane filter unit, achieving filtration while the platelets settle down, minimizing the contact area between the platelets and a filter built in the membrane filter unit, and finally reducing the quantity of the platelets adsorbed onto the membrane to obtain platelet rich plasma containing a maximum quantity of platelets.

In addition, the concentrated plasma can be readily gelated simply by repeating the filtration or adding calcium.

## Description

### TECHNICAL FIELD

The present invention relates to a device and method for extracting concentrated plasma. More particularly, the present invention relates to a device and method for extracting concentrated plasma that is designed to extract concentrated plasma from the blood plasma removed of red blood cells.

### BACKGROUND ART

In general, whole blood can be separated into blood corpuscles and blood plasma. The blood corpuscles fall into three general categories: red blood cells, white blood cells, and platelets. The blood plasma is mostly water, and contains clotting factors, electrolytes, and so forth.

To explain the blood plasma in further detail, the blood plasma consists of 60 to 85 % of water, 7 % of proteins, 1 % of fat, 0.1 % of sugar, and 0.9 % of other mineral ions. In this regard, the proteins in blood plasma can be classified into albumin and globulin, which are determined by using the difference in solubility in ammonium sulfate and individually consist of a combination of several proteins. The albumin constitutes about 55 % of the plasma protein and plays an important role in supplying proteins and maintaining the plasma colloid osmotic pressure. The globulin makes up about 38 % of the plasma protein and falls into one of the three categories: alpha globulin, beta globulin and gamma globulin. The alpha globulin contains lipoproteins and glycoproteins including fibrinogen and helps transport of vitamins, hormones, and so forth. The beta globulin performs a function as a carrier of prothrombin, plasma thromboplastin, iron, copper, or the like. The gamma globulin mostly contains immune antibodies.

The blood plasma, which plays a major role in the blood, can be injected into the human body to promote the transport of nutrient substances and thereby maximize its function such as inhibition of toxic substances.

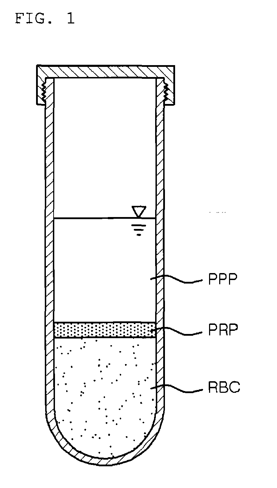

Therefore, as illustrated in FIG. 1, the whole blood (Wb) is separated into red blood cells (RBCs), platelet rich plasma (PRP) and platelet poor plasma (PPP). Generally, the red blood cells are separated from the whole blood, and platelet rich plasma (PRP) and platelet poor plasma (PPP) are extracted. The platelet rich plasma (PRP) and the platelet poor plasma (PPP) are used in various treatment and cosmetic applications, including the treatment of wounds including sinus elevation surgery for dental implant, heart surgery, orthopedic surgery, dermatological treatment, and so forth.

In addition, the platelet poor plasma (PPP) is separated from the whole blood and contains proteins and a small quantity of platelets not isolated yet. Therefore, a reisolation process for the platelet poor plasma (PPP) is performed to re-extract the proteins and platelets from the platelet poor plasma (PPP). For this purpose, the conventional method involves a repetition of centrifugal isolation and filtration on the platelet poor plasma (PPP).

However, even after the process of centrifugal isolation and filtration is done again on the platelet poor plasma (PPP) remaining after extraction of platelet rich plasma (PRP) from the blood plasma, the concentrated plasma re-extracted from the platelet poor plasma (PPP) has such a low content that it hardly contains a large quantity of proteins having a low molecular weight and not separated in the conventional process of centrifugal isolation and filtration. And, there is also a problem suggested in regards to the low efficiency of the conventional device and method that obtains concentrated plasma with low content.

### Prior Documents

### Patent Documents

(Patent Document 1) KR20100116160 10
(Patent Document 2) KR20100041436 10

### DISCLOSURE OF INVENTION

For solving the problems aforementioned, it is an object of the present invention to provide a device and method for extracting concentrated plasma that is designed to extract concentrated plasma with high concentration containing a maximum quantity of platelets while filtering the blood plasma out by passing the blood plasma removed of red blood cells through a filter by using a defined principle of pressure, gravity and vacuum.

To achieve the object of the present invention, there is provided a device for extracting concentrated plasma that includes: a membrane filter unit for isolating a component based on a predetermined particle diameter from blood plasma; and a first receiving member for providing a predetermined injection rate and a predetermined injection pressure to inject the blood plasma into the membrane filter unit and thereby to settle down platelets in the blood plasma.

The device further includes a pressurizing means mounted in connection to the first receiving member to provide the injection rate and the injection pressure of the blood plasma.

In this regard, the pressurizing means includes: a piston moving in connection to the membrane filter unit to inject the blood plasma in the first receiving member into the membrane filter unit; and a power source mounted to induce the movement of the piston.

The power source limits the moving distance of the piston to remain part of the blood plasma in the first receiving member for a predetermined period of time after the time that the blood plasma is completely injected into the membrane filter unit.

The power source maintains the piston at a moved position for a predetermined period of time after the time that the movement of the piston is completed.

The power source moves the piston in a reverse direction at a predetermined speed in order to separate platelets, fibrinogens, proteins, and so forth filled in the filter after completion of the filtration.

The pressurizing means further includes: an adaptor for intermediating a coupling of a first plunger mounted in the first receiving member and the piston to inject the blood plasma in the first receiving member into the membrane filter unit; and a controller for controlling the operation of the power source to regulate the injection rate and the injection pressure of the blood plasma according to the moving distance and the moving rate of the piston and the first plunger.

The pressurizing means further includes a direction-diverting switch for diverting the moving direction of the piston.

The membrane filter unit includes: a body having the first receiving membrane mounted thereon and including an inlet portion provided on either side portion of the membrane filter unit to exhaust air from the membrane filter unit when the blood plasma of the first receiving member flows in, and an outlet portion for exhausting a component having a particle diameter equal to or smaller than the predetermined particle diameter which has passed through the membrane filter unit; a hollow filter being mounted in the body and having a second space for receiving a component having a particle diameter equal to or smaller than the predetermined particle diameter which is filtered when the blood plasma of the first receiving member flows in through the inlet portion; and a first space formed between the hollow filter and the body to open into the outlet portion to temporarily receive a component having a particle diameter equal to or smaller than the predetermined particle diameter which has passed through the filter and to exhaust the same through the outlet portion.

In addition, the membrane filter unit further includes a fixing member mounted on the body for arranging the membrane filter unit in the upright position to settle down platelets of the blood plasma flowing into the membrane filter unit under the effect of gravity.

The device for extracting concentrated plasma according to the present invention further includes a second receiving member mounted on the membrane filter unit at a predetermined distance from the first receiving member to open into the second space 180 and receive air.

In this regard, the membrane filter unit further includes a valve mounted as an intermediate on a coupling portion between the inlet portion and the first and second receiving members. The valve is arranged to have the second space and the first receiving member open into each other at normal times. And, the valve is arranged to close the second space and the second receiving member when blood plasma is injected into the second space, and to open the second space into the second receiving member and inject air in the second receiving member into the second space when a component having a particle diameter equal to or larger than the predetermined particle diameter and remaining in the second space is to flow into the first receiving member.

On the other hand, according to the present invention, there is provided a method for extracting concentrated plasma that includes: a step S20 of injecting blood plasma into a membrane filter unit to settle down platelets in the blood plasma and thereby remaining a component having a particle diameter equal to or larger than the predetermined particle diameter which has not passed through a filter built in the membrane filter unit; and a step S40 of collecting a component having a particle diameter equal to or larger than the predetermined particle diameter and remaining in the membrane filter unit.

The method for extracting concentrated plasma according to the present invention further includes a step S10 of arranging the membrane filter unit in the upright position to reduce the contact area between the filter in the membrane filter unit and the platelets, before the filtering step S20.

The method for extracting concentrated plasma according to the present invention further includes a step S30 of waiting for a predetermined period of time after the blood plasma is completely injected and reducing pressure in the membrane filter unit to exhaust a component having a particle diameter equal to or smaller than the predetermined particle diameter.

The filtering step S20 includes injecting part of the blood plasma into the membrane filter unit. The first collecting step S40 includes collecting both a component having a particle diameter equal to or larger than the predetermined particle diameter which has passed through the filter and the remaining the blood plasma in one position.

The method for extracting concentrated plasma according to the present invention further includes a second collecting step S50 of injecting air into the membrane filter unit to collect again both the remaining component having a particle diameter equal to or larger than the predetermined particle diameter and the remaining blood plasma in one position.

In all the steps other than the collecting step, first and second spaces are formed by mounting a filter in a body of the membrane filter, where the second space holds a component having a particle diameter equal to or larger than the predetermined particle diameter which is filtered out from the filter when the blood plasma flows in, and the second space momentarily collecting a component having a particle diameter equal to or smaller than the predetermined particle diameter which has passed through the filter to exhaust the component through an outlet portion formed in the body of the membrane filter unit.

In addition, the filtering step S20 further includes a step S23 of injecting the blood plasma into the second space at a predetermined injection rate and a predetermined pressure to settle down the platelets. Here, the injection pressure of the blood plasma is determined to have the range of a load internal pressure imposed on the filter mounted in the membrane filter unit during the filtration is equal to or lower than the permissible internal pressure of the filter.

The injecting step S23 includes using a pressurizing means to move a first plunger mounted on a first receiving member holding the blood plasma and push the blood plasma. As a power source of the pressurizing means moves a piston to move the first plunger mounted on the piston, thereby injecting the blood plasma into the second space at a predetermined injection rate and a predetermined pressure. When part of the blood plasma is injected into the second space, the remainder of the blood plasma remains in the first receiving member. After filtration, the first plunger is moved back to collect a component having a particle diameter equal to or larger than the predetermined particle diameter and remaining in the second space into the first receiving member holding the remaining blood plasma.

Further, air in a second receiving member mounted on the membrane filter unit is injected into the second space to collect a component having a particle diameter equal to or larger than the predetermined particle diameter and remaining in the second space into the first receiving member holding the remaining blood plasma.

The first receiving member and the second receiving member are connected to the second space. Before the second collecting step S50, a valve is manipulated to have the second space open into the first receiving member and disconnect the second space from the second receiving member. In the second collecting step S50, the valve is manipulated to have the second space open into the first receiving member and the second receiving member.

In the filtering step S20, a valve 150 mounted on the opposite side of a first receiving member is closed to open into the membrane filter unit.

Therefore, in the filtering step S20, when the blood plasma is injected, an air layer is formed in part of the space of the membrane filter unit to limit the injection height of the blood plasma to a predetermined height or below.

The method for extracting concentrated plasma according to the present invention further includes a step S31 of completely closing the membrane filter unit and forming a vacuum pressure in the membrane filter, after the pressure-reducing step S30.

The collecting step S40 includes opening the valve to momentarily supply external air into the membrane filter unit and thereby collecting a component having a particle diameter equal to or larger than the predetermined particle diameter using the air.

In addition, the platelet rich plasma becomes gel by repeatedly filtering the blood plasma injected into the membrane filter unit or adding calcium and then repeatedly filtering the blood plasma.

### EFFECTS OF THE INVENTION

As described above, according to the present invention, the conventional process of separating platelet rich plasma and platelet poor plasma from the blood plasma can be omitted to simplify the process of extracting concentrated plasma which contains high concentration of platelets by passing the blood plasma removed of red blood cells through the membrane filter unit.

Further, the blood plasma can be passed through the membrane filter unit using a predetermined pressure and gravity for a sufficiently long period of time. This can allow the platelets in the blood plasma flowing into the membrane filter unit to settle down to the maximum, making it possible to perform the filtration while the platelets settle down, and minimize the contact area between the platelets and the filter built in the membrane filter unit. As a result, the quantity of the platelets adsorbed onto the membrane can be minimized, and the concentrated plasma can contain the maximum quantity of the platelets.

Furthermore, after the blood plasma is partly or completely injected into the membrane filter unit, the injection pressure is maintained for a predetermined period of time until the pressure in the membrane filter is sufficiently reduced. This can completely exhaust the component having a particle diameter equal to or smaller than the predetermined particle diameter and remaining in the membrane filter unit.

Moreover, the blood plasma is injected while the membrane filter unit is closed, so the injection height of the blood plasma can be limited to a predetermined height or below to prevent the contact between the blood plasma and the filter. This can minimize adsorption of the component in the blood plasma onto the filter.

In addition, the closed membrane filter unit is suddenly opened. This can flow away the component having a particle diameter equal to or larger than the predetermined particle diameter and remaining in the membrane filter unit momentarily, thereby readily releasing part of the components adsorbed onto the filter.

Further, the platelet rich plasma can be gelated by repeatedly filtering the blood plasma collected in the membrane filter unit or adding calcium to the blood plasma and then repeatedly filtering it.

### BRIEF DESCRIPTION OF DRAWINGS

The accompanying drawings, which are incorporated the specification, illustrate a preferred embodiment of the present invention and serve to explain the principles of the invention. It should be apparent that the accompanying drawings are not construed to limit the scope of the present invention. In the drawings:
FIG. 1 is a side diagram showing the whole blood under centrifugal separation.
FIG. 2 is a schematic constructional diagram showing a device for extracting concentrated plasma according to a preferred embodiment of the present invention.
FIG. 3 is a cross-sectional diagram showing the inside of the membrane filter unit shown in FIG. 2.
FIGS. 4a and 4b are operational diagrams of FIG. 2.
FIG. 4c is a constructional diagram showing that a second receiving member is excluded in the membrane filter unit of FIG. 2.
FIG. 5 is a flow chart showing a first extraction method for concentrated plasma using the extracting device of FIG. 2.
FIG. 6 is a flow chart showing a second extraction method for concentrated plasma using the extracting device of FIG. 2.

### BEST MODES FOR CARRYING OUT THE INVENTION

Hereinafter, a detailed description will be given as to the preferred embodiments of the present invention with reference to the accompanying drawings so as for those skilled in the art to readily carry out the present invention. But, in the detailed description of the operational principles regarding the preferred embodiments of the present invention, the specific description as to the known functions or constructions is to be excluded when it is considered to unnecessarily depart from the conception of the present invention.

### <Construction>

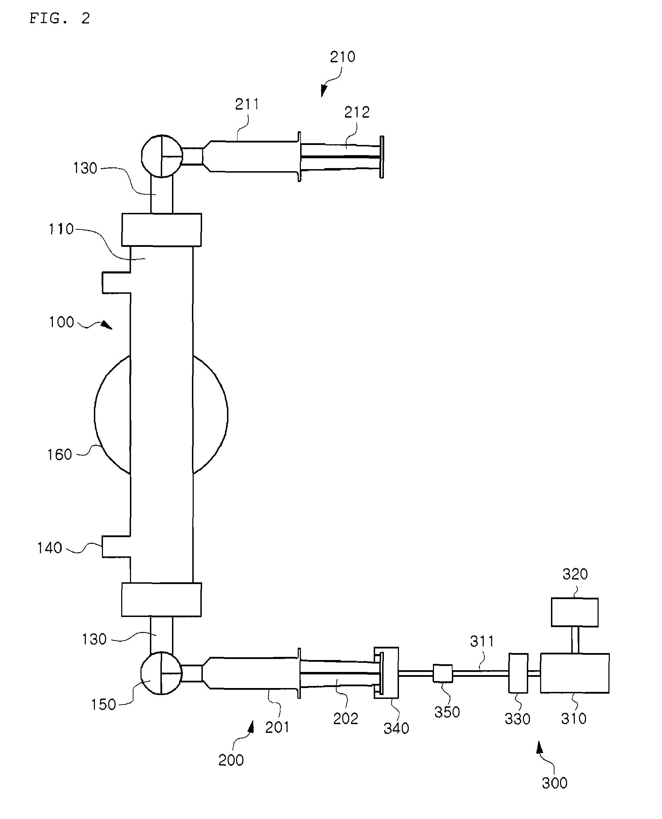

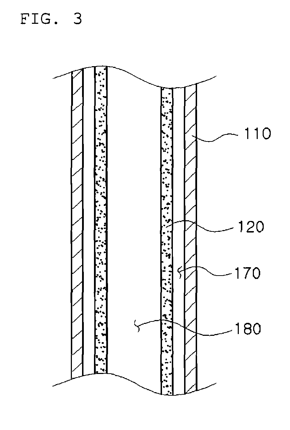

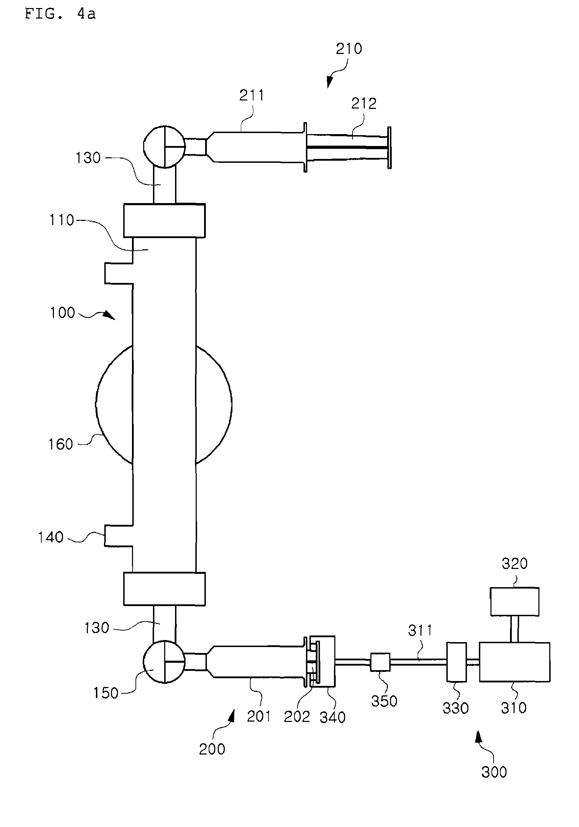

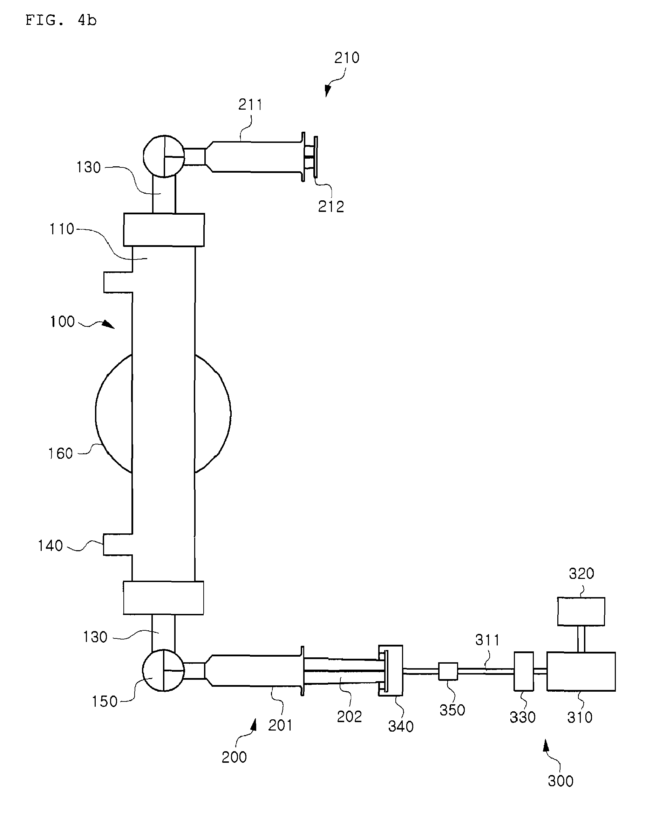

FIG. 2 is a schematic constructional diagram showing a device for extracting concentrated plasma according to a preferred embodiment of the present invention. FIG. 3 is a cross-sectional diagram showing the inside of the membrane filter unit shown in FIG. 2. FIGS. 4a and 4b are operational diagrams of FIG. 2.

The device for extracting concentrated plasma according to the present invention includes, as shown in FIG. 2, a membrane filter unit 100, a first receiving member 200, a second receiving member 210, and a pressurizing means 300.

The membrane filter unit 100 is a member for filtering blood plasma in order to exhaust a component having a particle diameter equal to or smaller than a predetermined particle diameter which is contained in the blood plasma. In this regard, the blood plasma used to extract platelet rich plasma in the present invention is preferably a blood plasma obtained by removing the whole blood of red blood cells through centrifugal separation, or it may be a platelet poor plasma generally available.

The membrane filter unit 100 includes a body 110, a filter 120 mounted in the body 110 and designed to have a component of a particle diameter equal to or smaller than the predetermined particle diameter which has passed through, an inlet portion 130 formed on either side portion of the body 110 and individually having the first and second receiving members 200 and 210, an outlet portion 140 mounted in the body 110 to exhaust the component of a particle diameter equal to or smaller than the predetermined particle diameter which has passed through the filter 120, a three-way valve 150 mounted to open/close the inlet portion 130, and a fixing member 160 for maintaining the body 110 in the upright position.

In this regard, the membrane filter unit 100 is to separate platelets, proteins, albumins, etc. from water, uric acid, potassium, phosphates, etc. as contained in the blood plasma. For separation, the cross-sectional area of the filter 120 is preferably about 0.04 m² to 0.08 m², more preferably about 0.06 m².

The membrane filter unit 100 may be an example of membrane filter unit, which is preferably used for blood osmosis. Of course, the membrane filter unit 100 may be a general filter capable of filtering out a component having a particle diameter equal to or larger than the predetermined particle diameter, such as platelets, proteins, albumins, etc. of the blood plasma, in addition to the membrane filter unit.

On the other hand, the filter 120 is built in the body 110, and the inlet portion 130 and the outlet portion 140 are formed on either side portion of the body 110. The body 110 preferably has a cylindrical shape, or it may have a polygonal pillar shape such as a rectangular pillar shape.

Between the inner wall of the body 100 and the filter 120 is formed a defined first space 170, which temporarily receives a component having a particle diameter equal to or smaller than the predetermined particle diameter which has passed through the filter 120. In other words, the first space 170 is an independent space disconnected from the inlet portion 130.

The filter 120 is a member through which a component having a particle diameter equal to or smaller than the predetermined particle diameter in the blood plasma is passed. The filter 120 has a hollow cylindrical shape or a hollow polygonal pillar shape that forms a second space 180 into which the blood plasma flows. In this regard, the second space 180 directly opens into the inlet portion 130 to have the blood plasma enter from the inlet portion 130. Accordingly, when the blood plasma flows into the second space 180, a component having a particle diameter equal to or smaller than the predetermined particle diameter in the blood plasma is passed through the filter 120 and flows into the first space 170, and a component having a particle diameter equal to or larger than the predetermined particle diameter in the blood plasma remains in the second space 180.

In this regard, there can be a modification of the embodiment in such a manner that the filter 120 has a panel shape and divides the inside of the body 110 into the first and second spaces 170 and 180, where the first space 170 opens into the outlet portion 140 and the second space 180 opens into the inlet portion 130.

In addition, the inlet portion 130 is formed on either side portion of the body 110. The first receiving member 200 receiving the blood plasma is mounted on the inlet portion 130 formed on the one side of the body 110, and the second receiving member 210 receiving air is mounted on the inlet portion 130 formed on the other side of the body 110. The inlet portion 130 is formed in direct connection to the second space 180 of the filter 120. This is not only to have the blood plasma of the first receiving member 200 flow directly into the second space 180 but also to have a component having a particle diameter equal to or larger than the predetermined particle diameter and remaining in the second space 180 flow into the first receiving member 200 by the pressure applied by the second receiving member 210. Other details in regards to the inlet portion 130 will be described later in association with the three-way valve 150.

In addition, the outlet portion 140 is formed on the one side of the body 100 or on either side of the body 100 to open into the first space 170. This is to exhaust a component having a particle diameter equal to or smaller than the predetermined particle diameter and flowing into the first space 170 through the filter 120.

The three-way valve 150 is mounted on either coupling portion of the inlet portion 130 and the first and second receiving members 200 and 210. The three-way valve 150 is operated to disconnect the first receiving member 200 and the second space 180 from the second receiving member 210 while the blood plasma of the first receiving member 200 is flowing into the second space 180 of the filter 100. Further, the three-way valve 150 is operated to have the second receiving member 210 open into the second space 180 and the first receiving member 200 so that, after the filtration of the blood plasma is completed, the air in the second receiving member 210 enters the second space 180 to flow a component having a particle diameter equal to or larger than the predetermined particle diameter and remaining in the second space 180 into the first receiving member 200.

The fixing member 160 is a member for maintaining the membrane filter unit 100 in the upright position, and it may be any general member designed so that the body 110 can be put in it, suspended from it, inserted into it or picked up from it. The fixing member 160 is excluded when the membrane filter unit 100 is used in a position other than the upright position. For this purpose, the membrane filter unit 100 is formed to have a wider bottom portion or to have a varied shape designed not to let off the blood plasma from the inlet portion 130 when the membrane filter unit having a cylindrical shape is laid on the side. In this case, there is no need of using the membrane filter unit in the upright position, if the platelets in the blood plasma settle down and have a minimum contact area with the filter 120.

In this regard, the purpose of using the fixing member 160 to fix the membrane filter unit 100 in the upright position is having the blood plasma flow in from the bottom portion of the body 110 and carrying out the filtration while the platelets in the blood plasma settle down under the effect of gravity. The settlement of the platelets minimizes the contact area between the platelets and the filter 120 to reduce the adsorption of the platelets onto the filter 120 to the minimum. This can minimize a loss of the platelets caused by the adsorption onto the filter 120 and render a maximum amount of platelets contained in the platelet rich plasma that is going to be extracted later.

The first receiving member 200 is a member for receiving the blood plasma, injecting it into the membrane filter unit 100 and collecting the filtered blood plasma, that is, platelet rich plasma. The first receiving member 200 includes a first receiving portion 201 for receiving the blood plasma or the platelet rich plasma obtained after the filtration and a first plunger 202 mounted to push the blood plasma in the first receiving portion 201 into the second space 180 of the membrane filter unit 100. The first receiving member 200 is preferably, for example, a syringe without a needle, and it may be constructed to include at least two separate members for receiving or pushing the blood plasma or receiving the concentrated plasma after the filtration.

On the other hand, the second receiving member 210 is a member for receiving air and injecting it into the second space 180 of the membrane 100 after the filtration to push a component having a predetermined particle diameter or a larger particle diameter remaining in the second space 180 into the first receiving member 200. The second receiving member 210 includes a second receiving portion 211 for receiving air and a second plunger 212 mounted to inject the air in the second receiving portion 211 into the second space 180 of the membrane filter unit 100. The second receiving member 210 is preferably, for example, a syringe without a needle, and it may be constructed to include at least two separate members for receiving or pushing air. In this regard, the mounting positions of the first and second receiving members 200 and 210 are the bottom portion and the top portion, respectively, and exchangeable with each other.

The pressurizing means 300 is a means for injecting the blood plasma in the first receiving member 200 into the second space 180 under a predetermined pressure, and includes a power source 310 having a piston 311 mounted to push or pull the first plunger 202. In addition to the power source 310, the pressurizing means 300 may further include a controller 320 mounted to control the operation of the power source 310, a decelerator 330 mounted on the power source 310, an adaptor 340 mounted to intermediate a coupling of the first plunger 202 and the piston 311, and a direction-diverting switch 350 mounted to divert the moving direction of the piston 311. In this regard, the piston 311 without the first plunger 202 may move by directly sliding inside the first receiving member 200.

First of all, the power source 310 is a device that enables the piston 311 coupled to the first plunger 202 of the first receiving member 200 to make a reciprocating movement in order to push or pull the first plunger 202. The power source 310 is preferably, for example, a motor. Otherwise, the power source 310 may also be a device with a separate piston 311 that enables the piston 311 to make a reciprocating movement.

In this regard, the pressure applied to inject the blood plasma into the membrane filter unit 100, that is, the pressure provided by the power source 310 may be equal to the permissible internal pressure of the filter 120 or below. For example, when the permissible internal pressure of the filter 120 is 500 mmHg, the load internal pressure of the filter 120 during the filtration is preferably in the range of about 300 to 450 mmHg.

In addition, the power source 310 provides an injection rate that allows the platelets in the blood plasma to settle down under the effect of gravity while the blood plasma is injected into the second space 180.

The controller 320 controls all the functions of the power source 310, including determination on whether to operate the power source 310 or not, the torque, that is, the magnitude of the pressure to inject the blood pressure into the second space 180, the running time, the moving distance of the piston 311, the moving direction of the piston 311, the stand-by time after movement of the piston 311, the injection pressure and the injection rate of the blood plasma, and so forth.

In one embodiment for extracting concentrated plasma from blood plasma, 5 portions of platelet rich plasma can be obtained by combining 3 portions of a component having a particle diameter equal to or larger than the predetermined particle diameter and remaining in the second space 180 and 2 portions of a blood plasma remaining in the first receiving member 200. For this purpose, the first plunger 202 in the first receiving member 200 moves only to the scale of 2 in the first receiving member 200 and then stops until a component having a particle diameter equal to or smaller than the predetermined particle diameter is completely exhausted through the outlet portion 140. The movement of the piston 311 in association with the movement of the first plunger 202 is under the control by the controller 320. It is of course possible to obtain 5 portions of the platelet rich plasma by injecting all the blood plasma of the receiving member 200 into the membrane filter unit 100 and filtering out a component having a particle diameter equal to or larger than the predetermined particle diameter and remaining in the second space 180.

In another embodiment for extracting concentrated plasma from blood plasma, 6 or 7 portions of platelet rich plasma can be obtained by combining 3 portions of the component having particle diameter equal to or larger than a predetermined particle diameter and remaining in the second space 180 and 3 or 4 portions of blood plasma remaining in the first receiving member 200. For this, the first plunger 202 moves only to the scale of 3 or 4 in the first receiving member 200 and then stops. The movement of the piston 311 is also under the control by the controller 320.

In other words, the controller 320 controls the movement of the piston 311 so that the quantity of the blood plasma remaining in the first receiving member 200 is regulated according to a desired amount of the component having a particle diameter equal to or larger than the predetermined particle diameter in the membrane filter unit 100.

Contrarily, the controller 320 can simply set the torque range of the power source 310 and control the power source 310 to operate within the range of the predetermined torque. In this case, each of the other functions of the power source 310 aforementioned can be activated independently or at least two of the other functions can be activated simultaneously, by way of separate switches or control buttons.

The decelerator 330 enhances the torque of the power source 310 and enables the stable occurrence of the torque.

The adaptor 340 is an intermediate for a stable coupling of the piston 311 and the first plunger 202. The one side of the adaptor 340 is firmly coupled to the first plunger 202, and the other side of the adaptor 340 is fixed to the piston 311.

The direction-diverting switch 350 is mounted to divert the moving direction of the piston 311 for injecting the blood plasma into the second space 180 or collecting a component having a particle diameter equal to or larger than the predetermined particle diameter and remaining in the second space 180 into the first receiving member 200 after the filtration. The direction-diverting switch 350 may be excluded, in which case the power source 310 can directly divert the moving direction of the piston 311. For example, when the power source 310 is a motor, it can divert the moving direction of the piston 311 by changing its rotational direction.

### <Examples>

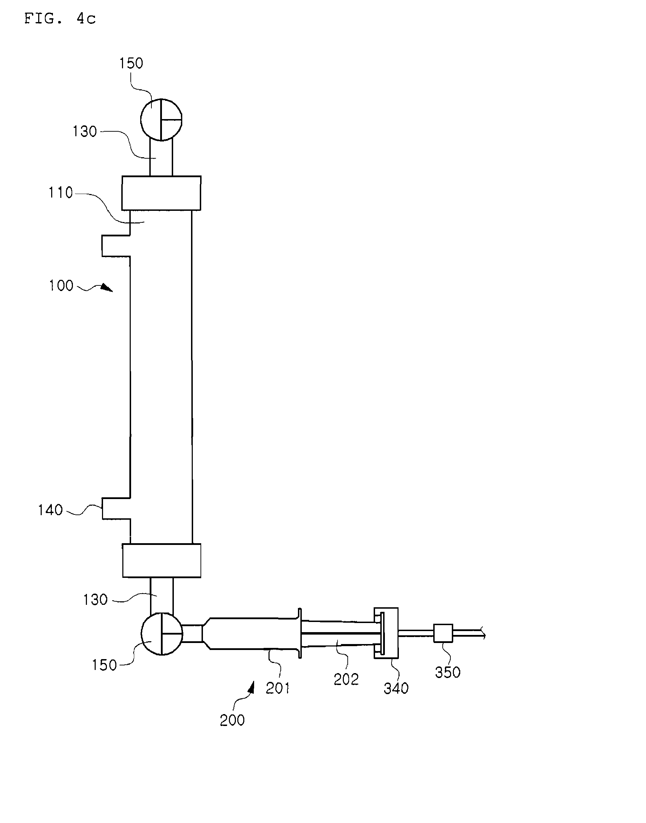

FIG. 4c is a constructional diagram showing the membrane filter unit of FIG. 2 without the second receiving member.

FIG. 4c is almost the same in construction as FIG. 2, excepting that the second receiving member 210 is excluded. Such a construction of FIG. 4c is to carry out a second method mentioned later, and its detailed description will be given in association with the description of the second method.

### <First Method>

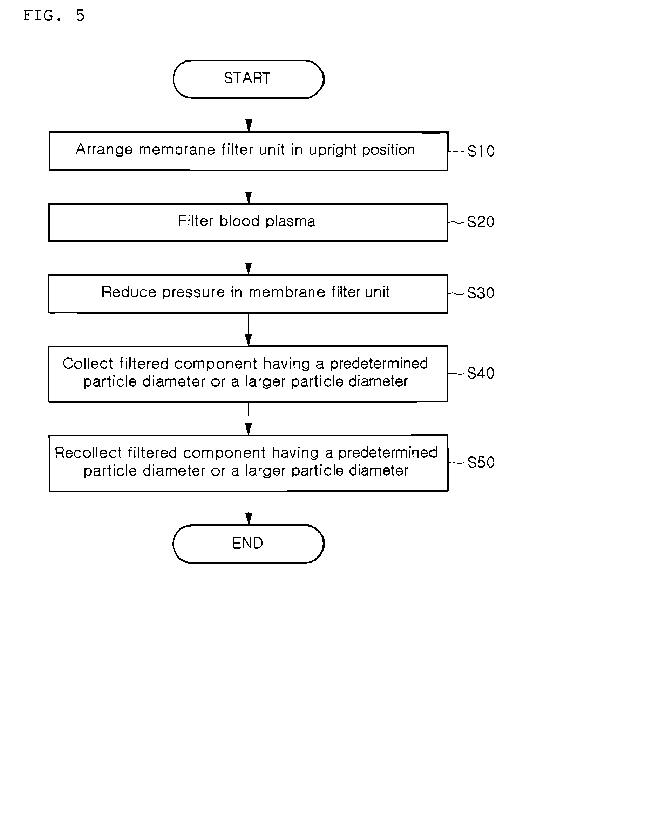

FIG. 5 is a flow chart showing a method for extracting concentrated plasma using the extracting device of FIG. 2.

Firstly, the membrane filter unit 100 is fixed in the upright position, in step S10. The arrangement is to make the platelets in the blood plasma settle down under the effect of gravity and then to filter the blood plasma. This can minimize the contact area between the platelets and the filter 120 and thereby reduce a loss of the platelets caused by the adsorption onto the filter 120.

Subsequently, the blood plasma is injected into the membrane filter unit 100 to undergo filtration, while allowing the platelets to settle down, in step S20. For this purpose, there is included a step S21 of receiving the blood plasma in the first receiving member 200 and then mounting the first receiving member 200 on the membrane filter unit 100. In this regard, the first receiving member 200 is mounted on the inlet portion 130 formed on the one side of the body 110 of the membrane filter unit 100, so it opens to the second space in the body 110. In this construction, the first receiving member 200 and the inlet portion 130 are coupled together via the three-way valve 150, which is manipulated to open or close the first receiving member 200 and the second space 180. For injecting the blood plasma into the second space 180, the three-way valve 150 can be manipulated to open the first receiving member 200 and the second space 180. Also, the first receiving member 200 and the second space 180 is kept open in order to collect the platelet rich plasma remaining in the second space into the first receiving member 200 after the subsequent filtration. Ultimately, a two-way valve 150 may be used in place of the three-way valve 150, and the first receiving member 200 may be mounted on the inlet portion 130 without using a valve.

For obtaining blood plasma, for example, 9 portions of whole blood is mixed with 1 portion of an anticoagulant, and the mixture is removed of red blood cells through centrifugal separation to extract about 4 to 5.5 portions of blood plasma. It is of course possible to extract a larger amount of the blood plasma by mixing a larger quantity of whole blood and a larger quantity of an anticoagulant and then preforming extraction of the blood plasma through centrifugal separation and removal of red blood cells.

Also, this step S20 may further include a step S22 of receiving air in the second receiving member 210 and then mounting the second receiving member 210 on the membrane filter unit 100. In this regard, the second receiving member 210 may be mounted on the inlet portion 130 formed on the other side of the body 110 via the three-way valve 150, which can be manipulated to open or close the second space 180. The mounting step S22 may be carried out right before the second collecting step S50 described later, or the second collecting step S50 may involve injecting external air into the second space 180, while the mounting step S22 is excluded.

When the blood plasma is injected into the second space 180, the three-way valve 150 is manipulated to open the inlet portion 130 of the second receiving member 210 to the exterior and thereby to exhaust the air of the membrane filter unit 100. In addition, when a component having a particle diameter equal to or larger than the predetermined particle diameter in the second space 180 is collected into the first receiving member 200 after the filtration, the second receiving member 210 is mounted on the inlet portion 130 and the three-way valve 150 is manipulated to close the second space 180 of the membrane filter unit 100 from the exterior and allow the second space 180 to open into the second receiving member 210. It is therefore possible to mount the second receiving member 210 and the inlet portion 130 without a two-way valve or another valve other than the three-way valve 150.

The step S20 may further include a step S23 of moving the piston 311 in one direction by using the pressurizing means 300, that is the power source 310 to move the first plunger 202 of the first receiving member 200 and thereby injecting the blood plasma into the second space 180. In this regard, the pressure that the pressurizing means 300 applies to inject the blood plasma into the second space 180 is in a predetermined pressure range equal to the permissible internal pressure of the filter 120 of the membrane filter unit 100 or below. In the case when the permissible internal pressure of the filter 120 is 500 mmHg, the load internal pressure of the filter 120 during the filtration is in the range of 300 mmHg to 450 mmHg, that is, approximately 60 to 90 %. And, the injection rate for the pressurizing means 300 injecting the blood plasma into the second space 180 is within the range that allows the platelets to settle down in the blood plasma injected in to the second space 180 and enables the continuous injection of the blood plasma while the platelets settle down. The injection pressure and the injection rate can be regulated under the control of the power source 310 and the decelerator 330. When the power source 310 is a motor, the decelerator 330 controls the motor velocity (rpm) of the motor to adjust the injection pressure and the injection rate.

When the injection pressure is 1,200 kg, for example, the pressure per unit area of the filter 120 in consideration of the gravity is 1,200kg/s² = 0.5kg/cm², which can be applied to calculate the injection pressure and the injection rate.

As the blood plasma is injected, the air in the membrane filter unit 100 is exhausted through the three-way valve 150 on the side of the second receiving member 210.

When the blood plasma is completely injected, the pressure in the membrane filter unit 100 is dropped in a predetermined period of time after the completion of the injection, in step S30. This can activate the settlement of the platelets more by reducing the pressure in the filter 120 of the membrane filter unit 100 and completely exhaust the component having a particle diameter equal to or smaller than the predetermined particle diameter which has passed through the filter 120 in the second space 180 and temporarily held in the first space 170 through the outlet portion 140.

In this regard, as a predetermined quantity of the blood plasma remains in the first receiving member 200 at the time when the injection of the blood plasma is completed, the first plunger 202 stops at a specific position. This is to remain a predetermined quantity of the blood plasma in the first receiving member 200. For extracting 5 portions of platelet rich plasma, for example, about 3 portions of the component having a particle diameter equal to or larger than the predetermined particle diameter which is obtained through filtration with the membrane filter unit 100 is mixed with about 2 portions of the blood plasma remaining in the first receiving member 200. Accordingly, a defined amount of the platelet rich plasma can be obtained by mixing a defined amount of the component having a particle diameter equal to or greater than the predetermined particle diameter which is obtained through filtration with the membrane filter unit 100 and a defined amount of the blood plasma remaining in the first receiving member 200. The amount of the blood plasma remaining in the first receiving member 200 can be determined based on the amount of the component obtained through the membrane filter unit 100, or the amount of the component obtained through the membrane filter unit 100 can be determined based on the amount of the blood plasma remaining in the first receiving member 200.

Contrarily, it is possible to obtain no more than the platelet rich plasma filtered through the membrane filter unit 100 by moving the first plunger 202 to a threshold moving position, stopping it and then injecting all the blood plasma of the first receiving member 200 into the membrane filter unit 100.

Of course, the moving and stop motions of the first plunger 202 are carried out by the movement of the piston 311 mounted on the first plunger 202, and the piston 311 is moved by the power source 310.

Subsequently, a component having a particle diameter equal to or larger than the predetermined particle diameter and remaining in the membrane filter unit 100 is first collected into the first receiving member 200, in step S40. In this regard, when the power source 310 forces the piston 311 to make a back motion and the first plunger 202 makes a back motion, most of the component having a particle diameter equal to or larger than the predetermined particle diameter in the second space 180 of the membrane filter unit 100 flows into the first receiving member 200. Here, the direction-diverting switch 350 is manipulated for the back motion of the piston 311.

Finally, air is injected into the membrane filter unit 100 to collect the remaining component having a particle diameter equal to or larger than the predetermined particle diameter into the first receiving member 200, in step S50. For this purpose, the three-way valve 150 of the second receiving member 210 is manipulated to make the second receiving member 210 open into the second space 180, and then the second plunger 212 is moved to inject the air in the second receiving member 210 into the second space 180, thereby forcing all the component having a particle diameter equal to or larger than the predetermined particle diameter and remaining in the second space 180 to flow into the first receiving member 200. It is of course possible to repeat the step S50 in order to completely flow the component having a particle diameter equal to or greater than the predetermined particle diameter into the first receiving member 200.

### <Second Method>

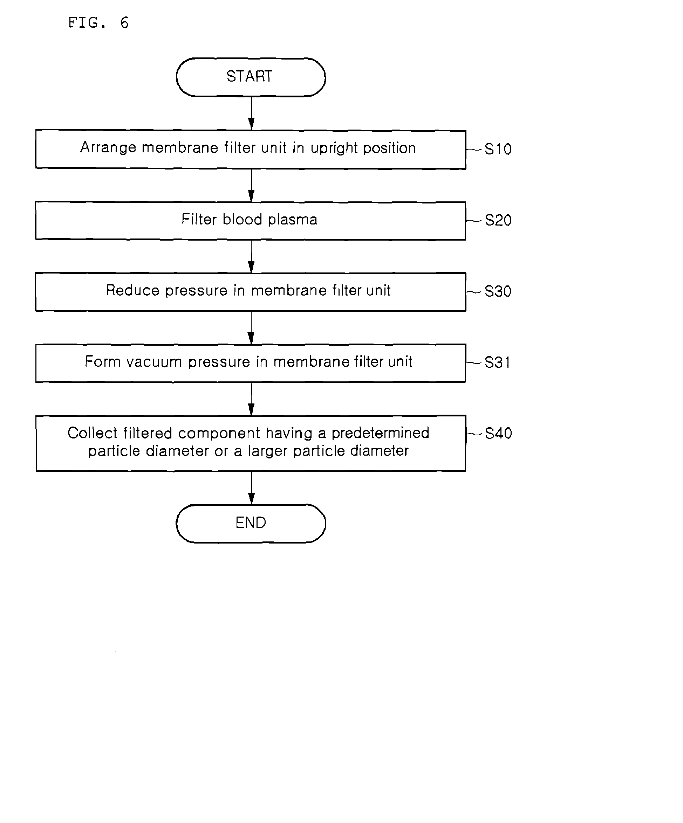

FIG. 6 is a flow chart showing a second extraction method for concentrated plasma using the extracting device of FIG. 2. The second method according to the present invention is a method of collecting a component having a particle diameter equal to or larger than the predetermined particle diameter without using the second receiving member 210. The second method is similar to the first method on the whole, except for a little difference in the steps. The steps of the second method the same as or similar to the steps of the first method are mentioned as the common content and described briefly under necessity.

Firstly, the membrane filter unit 100 is fixed in the upright position, in step S10. The step S10 is as defined in the first method.

Subsequently, the valve 150 mounted in the opposing side of the first receiving member 200 and open into the second space 180 is closed, and the blood plasma is injected into the membrane filter unit 100 and passed through the filter while the platelets are allowed to settle down, in step S20. As the valve 150 is closed, the second space 180 opens into the exterior through the outlet portion 140, so that the first plunger 202 of the first receiving member 200 is pressurized under the same pressure of the first method or the stronger pressure to inject the blood plasma into the second space 180. The valve 150 may be a three-way valve, but preferably a two-way valve because the second receiving member 210 is excluded.

Accordingly, the step S20 includes the step S21 of mounting the first receiving member 200 on the membrane filter unit 100 and the step S23 of moving the piston 311 in one direction by the power source 310 to move the first plunger 202 of the first receiving member 200 and thereby to inject the blood plasma into the second space 180, but it excludes the step S22 of mounting the second receiving member 210 on the membrane filter unit 100.

As the second space 180 is mostly closed, an air layer is formed on the opposite side to the first receiving member 200 in the second space 180 while the blood plasma is injected, and the air layer prevents the blood plasma from penetrating above a predetermined height. This can force the component having a relatively high force of adhesion such as platelets to remain below a predetermined height and limit the range of adsorbing the components of the blood plasma onto the filter 120 to the maximum.

Of course, the component having a particle diameter equal to or smaller than the predetermined particle diameter which has passed through the filter 120 is exhausted through the outlet portion 140 during the filtration.

Subsequently, it waits for a predetermined period of time after completion of the injection of the blood plasma and then the pressure in the membrane filter unit 100 is dropped, in step S30. The step S120 is also the same as defined in the first method.

Subsequently, the outlet portion 140 is blocked to close the second space 180 and then move the first plunger 202 of the first receiving member 200 back, thereby forming a vacuum pressure in the membrane filter unit 100, in step S31. In other words, when the outlet portion 140 is blocked, the second space 180 is completely closed, with the valve 150 closed. At this time, the power source 310 forces the piston 311 to make a back motion and moves the first plunger 202 back, so a strong vacuum pressure is formed in the membrane filter unit 100.

Finally, the valve 150 is momentarily opened to collect a component having a particle diameter equal to or larger than the predetermined particle diameter and remaining in the second space 180 into the first receiving member 200 all at once, in step S40. By part of the air momentarily introduced, the components adsorbed onto the filter 120 are released from the filter 120 and flow into the first receiving member 200.

This decompression method has an advantage that the components adsorbed onto the filter 120 during the filtration can be released from the filter 120 to the maximum. It is of course possible to repeat the steps S31 and S40 in a manual, semi-automatic, or automatic manner, in which case the operation of the valve 150 can be controlled through the controller in such a manner of controlling a solenoid valve.

The above-described first and second methods are applied to extract concentrated plasma from the blood plasma. In this regard, the concentrated plasma extracted according to the present invention has a platelet count about 4 to 5 times higher than the platelet rich plasma obtained by the conventional method using the centrifugal separation. A comparison between the concentrated plasma of the present invention and the whole blood is given in Table 1.

**[Table 1]**

| Whole blood | Platelet rich plasma |
|---|---|
| Platelet count | 4 to 11-fold |
| Protein count | 2 to 4-fold |
| Fibrinogen count | 3 to 5-fold |
| White blood cell count | 3 to 5-fold |
| Red blood cell count | 0.2 to 0.4-fold |

Particularly, the blood plasma is changed into a gel and used for many applications. In the conventional method of making the blood plasma containing an anticoagulant into a gel, bovine thrombin or collagen is added to the blood plasma. But, the bovine thrombin is a substance extracted from livestock and thus possibly contains a component harmful to the human body (e.g., mad cow disease factor, etc.).

Accordingly, the present invention repeats the filtration of the blood plasma using a membrane filter, more preferably adds calcium to promote the conversion of prothrombin into thrombin as the first step of the blood coagulation mechanism and thereby to form a large quantity of thrombin from the prothrombin in the blood plasma. Such a large quantity of thrombin serves as a factor to convert fibrinogens into fibrin monomers and is enough to form a large quantity of fibrin monomers. Finally, the fibrin monomers form fibrin clots and the platelet rich plasma is extracted in the form of a gel and thus can be used for various use purposes instead of fibrin glue.

As described above, those skilled in the art will appreciate that the present invention described herein is susceptible to variation other than those specifically described without changing its technical conception and essential features. Therefore, it should be understood that the foregoing description of preferred embodiments of the present invention has been presented for purposes of illustration and not intended to limit the scope of the present invention.

It should also be understood that the scope of the present invention is defined by the scope of the following claims other than the detailed description and construed to include all the variations and modifications derived from the meanings and scope of the claims and their equivalents.
100: membrane filter unit
110: body
120: filter
130: inlet portion
140: outlet portion
150: valve
160: fixing member
170: first space
180: second space
200: first receiving member
201: first receiving portion
202: first plunger
210: second receiving member
211: second receiving portion
212: second plunger
300: pressurizing means
310: power source
311: piston
320: controller
330: decelerator
340: adaptor
350: direction-diverting switch

## Claims

1. A device for extracting concentrated plasma, which is to extract concentrated plasma, the device comprising:
a membrane filter unit (100) for isolating a component based on a predetermined particle diameter from blood plasma; and
a first receiving member (200) for providing a predetermined injection rate and a predetermined injection pressure to inject the blood plasma into the membrane filter unit (100) and let thereby platelets in the blood plasma settle down by gravity.

2. The device for extracting concentrated plasma as claimed in claim 1, further comprising:
a pressurizing means (300) mounted in connection to the first receiving member (200) to provide the injection rate and the injection pressure of the blood plasma.

3. The device for extracting concentrated plasma as claimed in claim 2, wherein the pressurizing means (300) comprises:
a piston 311 moving relative to the membrane filter unit (100) to inject the blood plasma in the first receiving member (200) into the membrane filter unit (100); and
a power source (310) mounted to induce the movement of the piston (311).

4. The device for extracting concentrated plasma as claimed in claim 3, wherein the power source (310) limits the moving distance of the piston (311) to remain part of the blood plasma in the first receiving member (200) for a predetermined period of time after the time that the injection of the blood plasma into the membrane filter unit (100) is completed.

5. The device for extracting concentrated plasma as claimed in claim 3 or 4, wherein the power source (310) maintains the piston (311) at a position for the predetermined period of time after the time that the movement of the piston (311) is completed.

6. The device for extracting concentrated plasma as claimed in claim 3, wherein the pressurizing means (300) further comprises:
an adaptor (340) for intermediating a coupling of a first plunger (202) mounted in the first receiving member (200) and the piston (311) to inject the blood plasma in the first receiving member (200) into the membrane filter unit (100); and
a controller (320) for controlling the operation of the power source (310) to regulate the injection rate and the injection pressure of the blood plasma according to the moving distance and the moving rate of the piston (311) and the first plunger (202).

7. The device for extracting concentrated plasma as claimed in claim 3 or 6, wherein the pressurizing means (300) further comprises:
a direction-diverting switch (350) for diverting the moving direction of the piston (311).

8. The device for extracting concentrated plasma as claimed in claim 1, wherein the membrane filter unit (100) comprises:
a body 110 having the first receiving member (200) mounted thereon, the body comprising a first and a second inlet portion (130), wherein the air is exhausted through the second inlet portion (130) at one end of the membrane filter unit 100 when the blood plasma flows in through the first inlet portion (130) at the other end of the membrane filter unit (100), and an outlet portion (140) for exhausting a component having a particle diameter equal to or smaller than the predetermined particle diameter passed through the membrane filter unit (100);
a hollow filter (120) being mounted in the body (110) and having a second space (180) for receiving the component having a particle diameter equal to or smaller than the predetermined particle diameter which was filtered when the blood plasma of the first receiving member (200) flows in through the first inlet portion (130); and
a first space (170) formed between the hollow filter (120) and the body (110) to open into the outlet portion (140) to temporarily receive the component having a particle diameter equal to or smaller than the predetermined particle diameter which has passed through the filter (120) and exhaust the same through the outlet portion (140).

9. The device for extracting concentrated plasma as claimed in claim 1 or 8, wherein the membrane filter unit further comprises:
a fixing member (160) mounted on the body (110) for arranging the membrane filter unit in the upright position to settle down platelets of the blood plasma flowing into the membrane filter unit under the effect of gravity.

10. The device for extracting concentrated plasma as claimed in claim 8, further comprising:
a second receiving member (210) being mounted on the membrane filter unit (100) at a predetermined distance from the first receiving member (200) to open into the second space (180) and receiving air.

11. The device for extracting concentrated plasma as claimed in claim 10, wherein the membrane filter unit (100) further comprises a first valve (150) being mounted as an intermediate on a coupling portion between the first inlet portion (130) and the first receiving member (200) and a second valve (150) being mounted as an intermediate on a coupling portion between the second inlet portion (130) and the second receiving member (210),
the first valve being arranged to have the second space (180) and the first receiving member (200) open into each other at normal times, and
the second valve being arranged to close the second space (180) and the second receiving member (210) when blood plasma is injected into the second space (180), and to open the second space (180) into the second receiving member (210) and inject air in the second receiving member (210) into the second space (180) when a component having a particle diameter equal to or larger than the predetermined particle diameter and remaining in the second space (180) is to flow into the first receiving member (200).

12. A method for extracting concentrated plasma, which is to extract concentrated plasma, the method comprising:
a filtering step (S20) of injecting blood plasma into a membrane filter unit (100) to let the platelets settle down by gravity in the blood plasma and thereby remaining a component having a particle diameter equal to or larger than the predetermined particle diameter which has not passed through a filter (120) built in the membrane filter unit (100); and
a step (S40) of collecting the component having a particle diameter equal to or larger than the predetermined particle diameter and remaining in the membrane filter unit (100).

13. The method for extracting concentrated plasma as claimed in claim 12, further comprising:
a step (S10) of arranging the membrane filter unit (100) in the upright position to reduce the contact area between the filter (120) in the membrane filter unit (100) and the platelets, before the filtering step (S20).

14. The method for extracting concentrated plasma as claimed in claim 12, further comprising:
a step (S30) of waiting for a predetermined period of time after the blood plasma is completely injected and reducing pressure in the membrane filter unit (100) after the predetermined period of time to exhaust a component having a particle diameter equal to or smaller than the predetermined particle diameter.

15. The method for extracting concentrated plasma as claimed in claim 12, wherein the filtering step (S20) includes injecting part of the blood plasma into the membrane filter unit (100),
wherein the first collecting step (S40) includes collecting both a component, having a particle diameter equal to or larger than the predetermined particle diameter and remaining in the filter, and the remaining blood plasma in one position.

16. The method for extracting concentrated plasma as claimed in claim 15, further comprising:
a second collecting step (S50) of injecting air into the membrane filter unit (100) to collect again both the remaining component having a particle diameter equal to or larger than the predetermined particle diameter and the remaining blood plasma in one position.

17. The method for extracting concentrated plasma as claimed in claim 12, 14, 15, or 16, wherein in all the steps other than the collecting step, first and second spaces (170) and (180) are formed by mounting a filter (120) in a body (110) of the membrane filter (100), wherein the second space (180) holds a component having a particle diameter equal to or larger than the predetermined particle diameter which is filtered out from the filter (120) when the blood plasma flows in, the first space (170) temporarily collecting a component having a particle diameter equal to or smaller than the predetermined particle diameter which has passed through the filter (120) to exhaust the component through an outlet portion (140) formed in the body (110) of the membrane filter unit (100).

18. The method for extracting concentrated plasma as claimed in claim 17, wherein the filtering step (S20) further includes a step (S23) of injecting the blood plasma into the second space (180) at a predetermined injection rate and a predetermined pressure to let the platelets settle down by gravity,
wherein the injection pressure of the blood plasma is determined to have the range of a load internal pressure imposed on the filter (120) mounted in the membrane filter unit (100) during the filtration is equal to or lower than the permissible internal pressure of the filter (120).

19. The method for extracting concentrated plasma as claimed in claim 18, wherein the injecting step (S23) includes using a pressurizing means (300) to move a first plunger (202) mounted on a first receiving member (200) holding the blood plasma and push the blood plasma,
wherein as a power source (310) of the pressurizing means (300) moves a piston (311) to move the first plunger (202) mounted on the piston (311), thereby injecting the blood plasma into the second space 180 at a predetermined injection rate and a predetermined pressure,
wherein when part of the blood plasma is injected into the second space (180), the remainder of the blood plasma remains in the first receiving member (200),
wherein after filtration, the first plunger (202) is moved back to collect a component having a particle diameter equal to or larger than the predetermined particle diameter and remaining in the second space into the first receiving member (200) holding the remaining blood plasma.

20. The method for extracting concentrated plasma as claimed in claim 19, wherein air in a second receiving member (210) mounted on the membrane filter unit (100) is injected into the second space (180) to collect a component having a particle diameter equal to or larger than the predetermined particle diameter and remaining in the second space (180) into the first receiving member (200) holding the remaining blood plasma.

21. The method for extracting concentrated plasma as claimed in claim 20, wherein the first receiving member (200) and the second receiving member (210) are connected to the second space (180),
wherein before the second collecting step (S50), a first valve (150) is manipulated to have the second space (180) open into the first receiving member (200) and a second valve (150) is manipulated to disconnect the second space (180) from the second receiving member (210),
wherein in the second collecting step (S50), the first valve (150) is manipulated to have the second space (180) open into the first receiving member (200) and the second valve (150) is manipulated to have the second space (180) open into the second receiving member (210).

22. The method for extracting concentrated plasma as claimed in claim 14, wherein the filtering step (S20) includes opening a first valve (150) at a side of a receiving member (200) to have the second space (180) open into the first receiving member (200) and closing a second valve (150) mounted on the opposite side of the first receiving member (200).

23. The method for extracting concentrated plasma as claimed in claim 22, wherein in the filtering step (S20), when the blood plasma is injected, an air layer is formed in part of the space of the membrane filter unit (100) to limit the injection height of the blood plasma to a predetermined height or below.

24. The method for extracting concentrated plasma as claimed in claim 22, further comprising:
a step S31 of completely closing the membrane filter unit and forming a vacuum pressure in the membrane filter (100), after the pressure-reducing step (S30).

25. The method for extracting concentrated plasma as claimed in claim 24, wherein the collecting step S40 includes opening the second valve (150) to momentarily supply external air into the membrane filter unit (100) and thereby collecting a component having a particle diameter equal to or larger than the predetermined particle diameter using the air.

26. The method for extracting concentrated plasma as claimed in claim 12, wherein the concentrated plasma becomes gel by repeatedly filtering the blood plasma injected into the membrane filter unit (100) or adding calcium and then repeatedly filtering the blood plasma.
